# EUROPEAN PATENT APPLICATION

(11) **EP 2 042 132 A1**
(43) Date of publication of application: **01.04.2009**
(21) Application number: 08165308.1
(22) Date of filing: 26.09.2008
(51) Int. Cl.: A61F 2/38

(54) **Tibial prosthesis comprising a mobile bearing**

(30) Priority: 22.11.2007 GB 0722905; 27.09.2007 GB 0718856
(71) Applicant: Finsbury (Development) Limited, Leatherhead Surrey KT22 7BA (GB)
(72) Inventor: Webb, Adrian, Leatherhead, Surrey KT22 7GF (GB); Tuke, Michael Anthony, Guildford, Surrey GU1 3TF (GB)
(74) Representative: Smaggasgale, Gillian Helen

(57) **Abstract**

A mobile bearing tibial implant comprising:
a tibial component comprising: a tibial plate having a proximal face and a distal face; a tibial post extends from the distal face of the tibial plate which in use will anchor the tibial plate to the proximal end of a resected tibia; and a generally rectangular tibial projection extending upwardly from the proximal face of the tibial plate, the central longitudinal plane of the said tibial projection being co-linearly located on the sagittal plane passing substantially through the centre of the knee; and
a meniscal bearing component having a substantially flat distal face for glidable seating on the tibial plate and a proximal face configured to receive in use an articulating condylar surface of a femoral component, the meniscal component having a recess therein opening on the distal face of the component, said recess being shaped to receive the tibial projection and to allow the meniscal bearing component to rotate about a centre of rotation passing through the tibial projection but to prevent or limit anterior/posterior translation of the meniscal bearing component on the tibial plate.

## Description

The present invention relates to a knee prostheses. More particularly it relates to a tibial implant. Still more particularly, it relates to a mobile bearing tibial implant.

The knee joint is formed by the distal end of the femur, the proximal end of the tibia and a meniscus located therebetween. A plurality of ligaments not only hold these components in the correct alignment but also allow them to move relative to one another as the knee is flexed and tensed. These ligaments also allow for some lateral and medial rotation. Since the knee supports nearly the entire weight of the body, and is subjected to various stresses as the body moves, it is vulnerable to damage through injury or through the development of osteoarthritis.

When the knee has become damaged, the mobility of the body can be severely compromised. Various prostheses have therefore been suggested to replaced the damaged natural joint. Whilst hinged components have been suggested it was realised that components which mimic the structure of the natural knee would be more appropriate.

Initial prostheses of this kind comprised a femoral component for location on the resected distal end of the femur and a tibial component for location on the proximal end of the resected proximal end of the tibia. Whilst these allowed for some articulation, the range of motion and in particular the rotation of a healthy natural joint was not achieved. With time, improved prosthesis have been suggested to address this problem.

For example, a so-called total knee prosthesis has been produced which comprises a tibial plate and a femoral component with an intervening meniscal bearing component which may have medial and lateral sides. Typically the tibial plate and femoral compartment are made from a suitable metal or metal alloy, such as an alloy of cobalt and chromium, whereas the meniscal bearing component is made from a synthetic plastics material, for example ultra high molecular weight polyethylene. In some designs the meniscal bearing component is fixed to the tibial plate. However, in others it is free to float to some extent with respect to the tibial plate in an attempt to mimic more closely the natural movement of the knee. In some arrangements the meniscal component may be allowed rotary and/or sliding motion on the tibial plate. This movement may reduce the occurrences of dislocation of the components of the prosthesis in normal walking.

Examples of prior art prosthesis which include some floating bearing surfaces include US 6972039, US2005/0209702, US6428577, US6413279, US6319283, US 6296666 and US6238434. Whilst these and other prior art prostheses offer advantages over earlier arrangements in providing certain degrees of freedom that are not present where the bearing surface is fixed to the tibial plate or is integral therewith, problems with dislocation can still occur, particularly if the ligaments salvaged and tensioned by the surgeon during the implantation of the prosthesis do not provide the required support. Further, where the meniscal component is allowed to have anterior-posterior translation motion, spinout of the bearing may occur.

With a view to increasing stability and to reduce the risks of spinout, prosthesis have been suggested which include a post extending superiorly from the tibial component and which in use will be located in the inter-condylar space of the femur. However, these prosthesis suffer from various disadvantages. In particular, the interaction between the surface of the post and the surface of the femoral component of the prosthesis can lead to metal wear which may lead to metal particles being released which in turn can lead to accelerated wear of any polymeric components possibly requiring early revision operations.

It is therefore desirable to provide a mobile bearing tibial implant comprising a tibial component and a meniscal component which when used together provide the desired range of motion in flexion, tension and in rotation and which overcome the problem associated with the risk of spinout noted in the prior art. It is further desirable to provide an arrangement which has non metal on moral components which would lead to metal on metal wear.

Thus according to the present invention there is provided a mobile bearing tibial implant comprising:
a tibial component comprising: a tibial plate having a proximal face and a distal face; a tibial post extends from the distal face of the tibial plate which in use will anchor the tibial plate to the proximal end of a resected tibia; and a generally rectangular tibial projection extending upwardly from the proximal face of the tibial plate, the central longitudinal plane of the said tibial projection being co-linearly located on the sagittal plane passing substantially through the centre of the knee; and
a meniscal bearing component having a substantially flat distal face for glidable seating on the tibial plate and a proximal face configured to receive in use an articulating condylar surface of a femoral component, the meniscal component having a recess therein opening on the distal face of the component, said recess being shaped to receive the tibial projection and to allow the meniscal bearing component to rotate about a centre of rotation passing through the tibial projection but to prevent or limit anterior/posterior translation of the meniscal bearing component on the tibial plate.

By "substantially through the centre of the knee" we mean that the tibial projection may be located on the plane passing through the centre of the knee or one offset therefrom but which provides the desired product. Thus the plane may be at or near to the centre of the knee.

Where the mobile bearing tibial implant of the present invention is used to replace the proximal end of a natural tibia, the patient can expect, after knee arthroplasty, to have a satisfactory range of movement in the joint and will have a significant reduction in the risk of dislocation and spinout that would have been expected with prior art prostheses.

The tibial plate and the tibial post may be formed as a single piece or in one alternative arrangement, the tibial post may be a separate component connectable to the tibial plate. The tibial projection may be formed as a single piece with the tibial plate or in one alternative arrangement, the tibial projection may be a separate component connectable to the tibial plate.

Thus in one arrangement the tibial projection, the tibial plate and the tibial post are all formed as a single piece. In one further arrangement, the tibial post itself or a fastening therefrom may pass through an aperture in the tibial plate to engage with the underside of the tibial projection and thereby lock the three elements together. In a still further arrangement, a fastening means may extend downwardly from the tibial projection, pass through an aperture in the tibial plate and then engage with the tibial post thereby locking the three components together. In the latter two arrangements, the fastening means may be integral with the post or projection or in an alternative arrangement may be a separate component such as a bolt.

Means may be included to prevent the tibial plate from rotating on the resected tibia. In use, the tibial post will be inserted into a well drilled into the resected tibia. Although the post will be a tight fit in the well and will generally be held in place using bone cement, the rotational forces on the plate in use can be significant. In order to counteract these and to prevent the risk of the post being loosened in the well, means may be provided to prevent rotation of the plate. One example of suitable means to prevent rotation are anchoring pins which extend downwardly from the distal face of the tibial plate and which can be inserted into the bone. In one arrangement, two anchoring pins are provided one of which is located to the left of the tibial post and the other to the right thereof.

The tibial projection will be located on or near the sagittal plane passing through the centre of the tibial plate and will be aligned such that it is generally longitudinally centered on or near to that plane. In one arrangement, the anterior and posterior ends of the generally rectangular tibial projection may be of any suitable size. In one arrangement it may have a length in the region of about 17.5mm to about 22 mm. The width of the generally rectangular tibial projection may be in the region of about 11mm. However, the size selected will generally depend on the tray size.

The anterior and posterior ends of the generally rectangular tibial projection may be curved outwardly from the projection. The radii of the arcs may be the same or different. In a preferred arrangement, the posterior radius will be centered around the axis of rotation. The anterior radius will not generally be tangent to the sides of the projection. Where the radii of the anterior and posterior arcs are different, the centre of the posterior radius may be the centre of the anterior radius. In a particularly preferred arrangement, the overall length of the tibial projection will define the anterior radius. In one arrangement, only the posterior end will be curved.

The recess located in the underside of the meniscal bearing component will be of any suitable size to allow the meniscal bearing component to rotate about a centre of rotation passing through the tibial projection but to prevent anterior/posterior translation of the meniscal bearing component on the tibial plate. It will be in the underside of the meniscal bearing component and does not communicate with the proximal surface thereof. In one arrangement, the recess will have a tear drop cross section such that an anterior portion thereof has a longer arc length than an anterior portion of the tibial projection. In general the posterior and anterior radii of the recess will correspond to those of the post. The posterior wall of the recess will have a smaller arc length than the anterior wall of the recess but will be of substantially the same size and configuration as the posterior portion of the tibial projection. The posterior wall of the recess will be sized to be a sliding fit with the corresponding end of the tibial projection such that in use the bearing will rotate about a centre of rotation passing through the tibial projection. In a particularly preferred arrangement, the recess will have two angled faces tangent to the posterior radius and which define the limit of rotation of the bearing.

The size of the recess will allow the required range of rotation. In one arrangement it will allow from about 0° to about 30° rotation in each direction from the central position. In a preferred arrangement, the present invention will allow about 5° to about 30° in each direction from the central position. In a further arrangement, it will allow about 15° clockwise and about 15° anti-clockwise rotation.

The tibial plate, post and projection are preferably formed from a metal material and are more preferably from a cobalt chrome alloy. The meniscal bearing component is preferably formed from a polymeric material. In a preferred arrangement, it will be formed from an ultra high molecular weight polyethylene.

Whilst the proximal face of the articulating condylar surface is described as being configured to receive in use an articulating condylar surface from a femoral component, it will be understood that whilst the femoral component will generally be a prosthetic femoral component it could be the distal end of a femur.

The present invention will now be described by way of example with reference to the accompanying drawings in which:
- Figure 1: is an exploded isometric view of one embodiment of the present invention;
- Figure 2: is the view of Figure 1 illustrating hidden details;
- Figure 3: is an assembled isometric view of the prosthesis of the present invention;
- Figure 4: is a section view of Figure 3 from the side;
- Figure 5: is a section view from above with the bearing component centered on the tibial tray;
- Figure 6: is a section view from above illustrating anticlockwise rotation;
- Figure 7: is a section view from above illustrating clockwise rotation;
- Figure 8: is a section view from the side of an alternative arrangement of the present invention;
- Figure 9: is an exploded view of the components of Figure 8
- Figure 10: is a section view from the side of a further alternative arrangement of the present invention; and
- Figure 11: is an exploded view of the components of Figure 10.

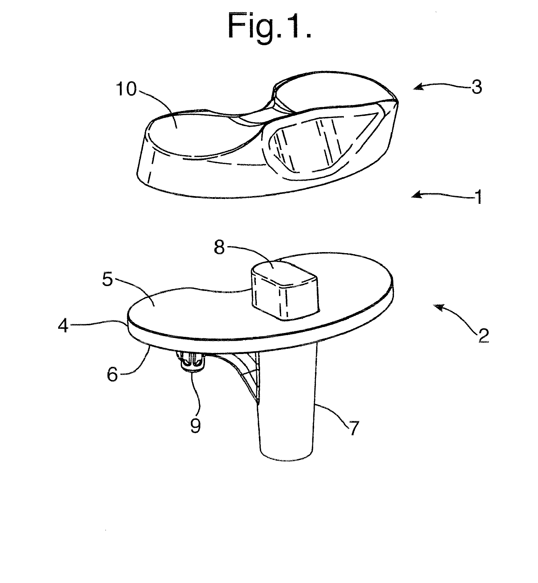

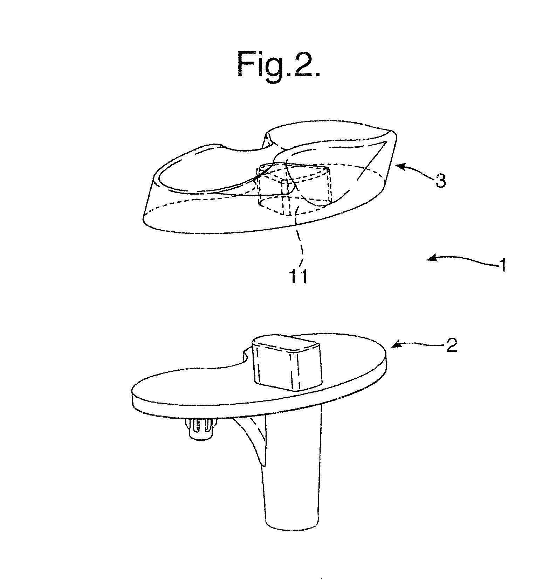

As illustrated in Figures 1 and 2, the mobile bearing tibial implant 1 of the present invention comprises a tibial component 2 and a meniscal bearing component 3. The tibial component 2 comprises a tibial plate 4 having a proximal face 5 and a distal face 6. A tibial post 7 extends downwardly from the distal face 6 of the tibial plate 4. In use this post will be located in a well in the resected tibia. Anchoring pins 9 are also provided extending form the distal face 6 of the tibial plate. The pin 9 located to the left of the post 7 can be seen in Figure 1, a second pin (not shown) will be located to the right of the post 7. A tibial projection 8 extends upwardly from the proximal face 5.

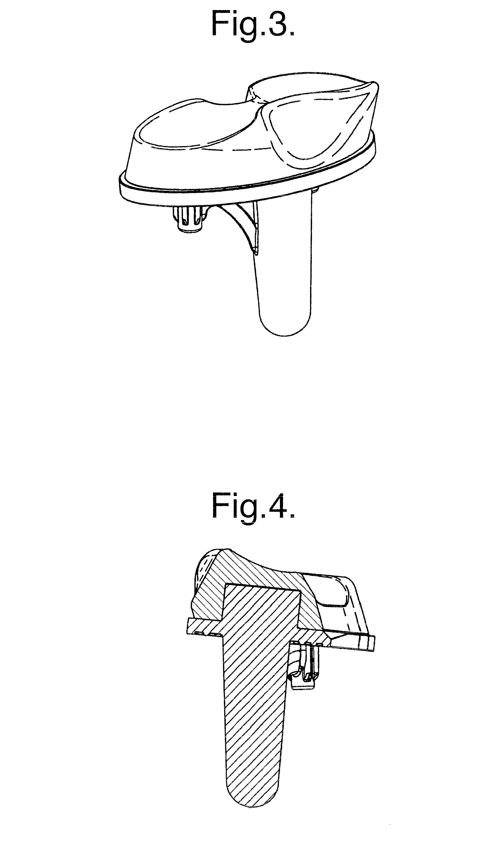

The meniscal bearing component 3 has a bearing surface 10 shaped to provide an articulating surface for a femoral component. A recess 11 is located in the underside of the bearing component. The recess does not pass through the bearing and therefore does not communicate with the bearing surface 10. The assembled implant is illustrated in Figures 3 and 4.

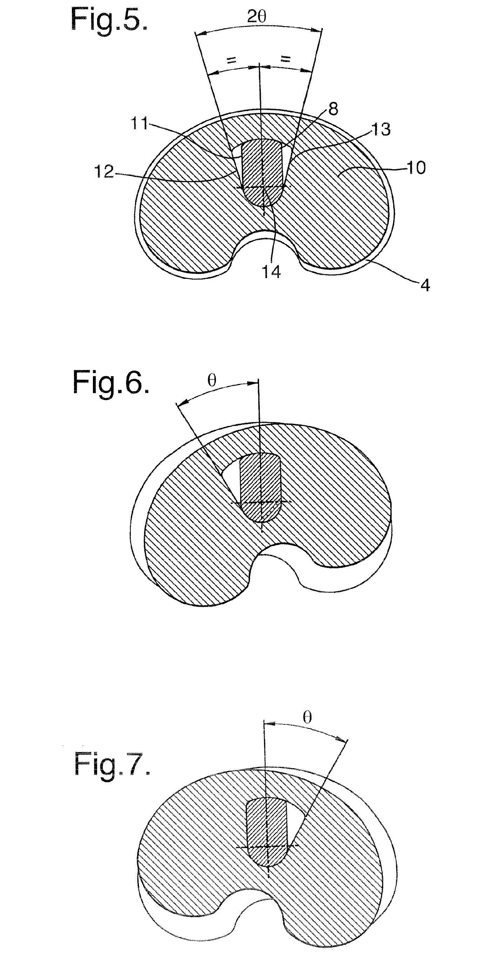

As illustrated in the sectional views of Figures 5 to 7. The tibial projection 8 is of generally rectangular configuration although the anterior and posterior ends are curved. The recess 11 is of a generally teardrop shape and has walls 12 and 13 which provide stops to the rotation of the projection 8 in the recess 11. The length of the walls 12, 13 correspond to the length of the tibial projection 8 such that anterior-posterior movement of the meniscal bearing component 11 on the tibial plate 4 is prevented.

The relative configurations of the tibial projection 8 and the recess 11, allow the meniscal bearing component 3 to rotate about the centre of rotation 14. Rotation allowed in the anticlockwise and clockwise directions are preferably the same and may be about 15° in each

direction.

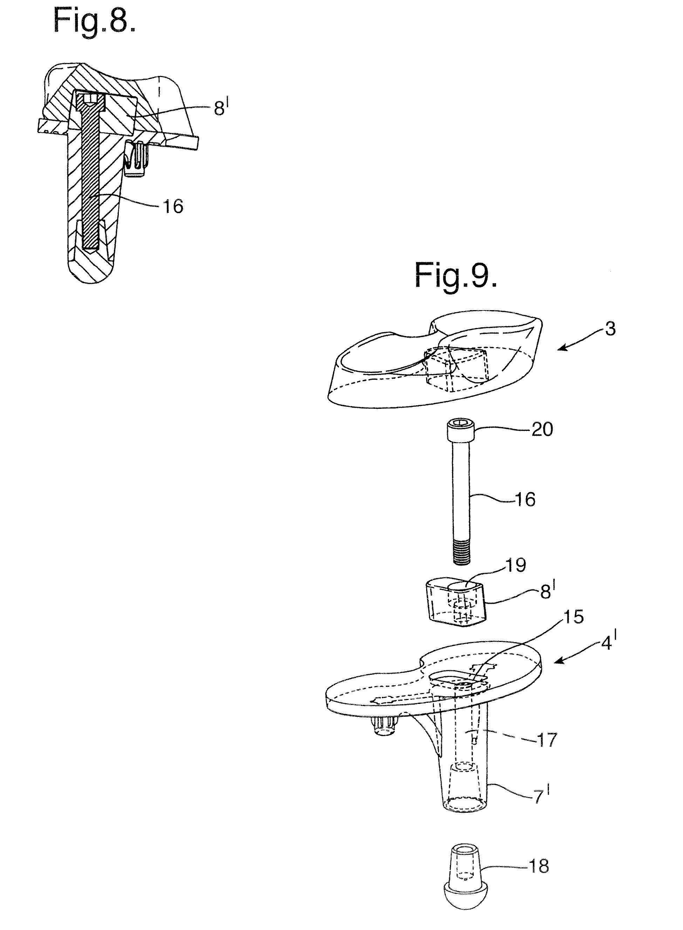

An alternative arrangement is illustrated in Figures 8 and 9. In this arrangement, the tibial projection 8' is a separate component form the tibial plate 4'. A shallow trough 15 may be provided in the proximal surface of the tray 4' into which the tibial projection 8' can sit. A fastening means 16 in the form of a bolt is passed through the tibial projection 8' and through a bore 17 in the tibial post 7'. An aperture 19 will be provided in the upper surface of the tibial projection to accommodate the head 20 of the bolt such that its presence will not prevent the movement of the meniscal bearing component on the tibial tray. In the illustrated arrangement a locking means 18 is provided which can be inserted into the bore 17 and will have an internal screw thread which will lock with bolt 16. In an alternative arrangement, the locking means may be integral with the tibial post 7'. The meniscal bearing component will sit on the tibial projection as in the above embodiment.

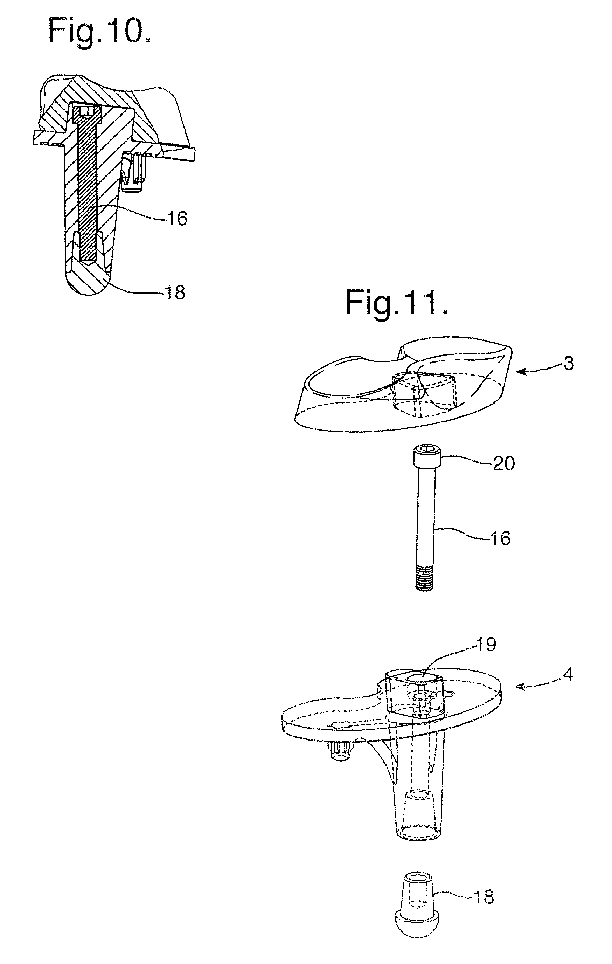

A further alternative arrangement is illustrated in Figures 10 and 11. This is similarto the arrangement illustrated in Figures 8 and 9 except that the tibial projection is integrally formed with the tibial plate.

## Claims

1. A mobile bearing tibial implant comprising:
a tibial component comprising: a tibial plate having a proximal face and a distal face; a tibial post extends from the distal face of the tibial plate which in use will anchor the tibial plate to the proximal end of a resected tibia; and a generally rectangular tibial projection extending upwardly from the proximal face of the tibial plate, the central longitudinal plane of the said tibial projection being co-linearly located on the sagittal plane passing substantially through the centre of the knee; and
a meniscal bearing component having a substantially flat distal face for glidable seating on the tibial plate and a proximal face configured to receive in use an articulating condylar surface of a femoral component, the meniscal component having a recess therein opening on the distal face of the component, said recess being shaped to receive the tibial projection and to allow the meniscal bearing component to rotate about a centre of rotation passing through the tibi al projection but to prevent or limit anterior/posterior translation of the meniscal bearing component on the tibial plate.

2. A mobile bearing tibial implant according to Claim 1 wherein the tibial plate and the tibial post are formed as a single piece.

3. A mobile bearing tibial implant according to Claim 1 wherein the tibial post is a separate component connectable to the tibial plate.

4. A mobile bearing tibial implant according to any one of Claims 1 to 3 wherein the tibial projection is formed as a single piece with the tibial plate.

5. A mobile bearing tibial implant according to any one of Claims 1 to 3 wherein the tibial projection is a separate component connectable to the tibial plate.

6. A mobile bearing tibial implant according to any one of Claims 1 to 5 wherein means are included to prevent the tibial plate from rotating on the resected tibia.

7. A mobile bearing tibial implant according to Claim 6 wherein the means to prevent the rotation of the tibial plate are two anchoring pins one of which is located to the left of the tibial post and the other to the right thereof.

8. A mobile bearing tibial implant according to any one of Claims 1 to 7 wherein the anterior and posterior ends of the generally tibial projection are arcs curved outwardly from the projection.

9. A mobile bearing tibial implant according to Claim 8 wherein the radii of the arcs are different.

10. A mobile bearing tibial implant according Claims 8 to 9 wherein the posterior radius is centered around the axis of rotation.

11. A mobile bearing tibial implant according to Claim 9 wherein the centre of the posterior radius is the centre of the anterior radius.

12. A mobile bearing tibial implant according to any one of Claims 1 to 11 wherein the overall length of the tibial projection defines the anterior radius.

13. A mobile bearing tibial implant according to any one of Claims 1 to 12 wherein the recess has a tear drop cross section.

14. A mobile bearing tibial implant according to Claim 13 wherein the posterior and anterior radii of the recess correspond to those of the post.

15. A mobile bearing tibial implant according to any one of Claims 1 to 14 wherein the recess has two angled faces tangent to the posterior radius and which define the limit of rotation of the bearing.

16. A mobile bearing tibial implant according to any one of Claims 1 to 15 wherein the size of the recess allows from about 5° to about 30° rotation in each direction from the central position.

17. A mobile bearing tibial implant according to any one of Claims 1 to 15 wherein the size of the recess allows from about 15° rotation in each direction from the central position.
